# EUROPÄISCHE PATENTSCHRIFT

(11) **EP 0 515 597 B1**
(45) Veröffentlichungstag und Bekanntmachung des Hinweises auf die Patenterteilung: **21.08.1996**
(21) Anmeldenummer: 91920319.0
(22) Anmeldetag: 16.11.1991
(51) Int. Cl.: A61M 39/00, F16K 15/14

(54) **RÜCKSCHLAGVENTIL, INSBESONDERE FÜR MEDIZINISCHE INFUSIONSGERÄTE**
NONRETURN VALVE, IN PARTICULAR FOR MEDICAL INFUSION APPLIANCES
CLAPET DE NON-RETOUR, NOTAMMENT POUR APPAREILS MEDICAUX DE PERFUSION

(30) Priorität: 13.12.1990 DE 4039814
(43) Veröffentlichungstag der Anmeldung: 02.12.1992
(73) Patentinhaber: Forberg, Hans-Jürgen, D-23738 Damlos (DE)
(72) Erfinder: Forberg, Hans-Jürgen, D-23738 Damlos (DE)
(74) Vertreter: Vollmann, Heiko, Dipl.-Ing.
(86) Internationale Anmeldenummer: DE9100896
(87) Internationale Veröffentlichungsnummer: WO9210232

(56) Entgegenhaltungen:
- EP-A- 0 333 305
- DE-A- 2 603 712
- DE-U- 8 805 638
- FR-A- 2 142 846

## Beschreibung

Die Erfindung gebt aus von einem Rückschlagventil, insbesondere für medizinische Geräte, nach dem Oberbegriff des Patentanspruches 1.

Ein derartiges Rückschlagventil ist in der FR-A-21 42 846 beschrieben. Es besteht aus einem Gehäuse mit einer durch eine Zwischenwand in zwei Teilräume unterteilten Kammer, deren einer Teilraum mit einem Flüssigkeitseinlaß und deren anderer Teilraum mit einem Flüssigkeitsauslaß versehen ist, und aus einer den Flüssigkeitsdurchfluß durch die Kammer freigebenden und unterbrechenden Membran. Die Membran besitzt einen zentralen Dichtungsteil, der über eine gerade, schräge und steife Verbindungswand mit einem in dem Gehäuse einklemmbaren Randteil der Membran verbunden ist. Der zentrale Dichtungsteil besitzt vorstehende, ringförmige Dichtungsstellen, die normal unter ventilschließender Vorspannung an zum Teil zurückspringenden Dichtungsflächen der Zwischenwand anliegen.

In der DE-A-26 03 712 ist ein Rückschlagventil beschrieben, das mit Ausnahme der Dichtungsmembran und der Zwischenwand im wesentlichen in seinem Aufbau demjenigen nach dem vorgenannten Dokument gleicht. Die Membran ist ein scheibenförmiges, gleichbleibend dickes Element mit zentralem Strömungsdurchlaß, das in seinem Randbereich in dem Gehäuse eingeklemmt ist und in seinem Mittenbereich unter Vorspannung an einem ringförmigen Dichtungsvorsprung des einen Gehäuseteiles anliegt, weil eine Zwischenwand fehlt.

Ein weiteres Rückschlagventil ist in der EP-A-02 47 824 beschrieben Dieses Ventil ist für geringe Druckdifferenzen zwischen seiner Zuströmseite und seiner Abströmseite nicht geeignet, d. h. ein sicheres Öffnen und Schließen des Ventils ist bei geringen Druckdifferenzen nicht gewährleistet. Um überhaupt ein Funktionieren bei kleinen Druckdifferenzen zu erreichen, darf die Vorspannkraft der eingespannten Membran bei diesem Ventil nur sehr gering sein. Diese Bedingung ist aber nur sehr schwer einzuhalten, und zwar im wesentlichen wegen der bei diesem Ventil erforderlichen engen Fertigungstoleranzen bei der Herstellung der Ventilteile, wobei sich schon kleine Abweichungen verhältnismäßig stark nachteilig auswirken. Kleine Fertigungstoleranzen bedingen aber höhere Fertigungskosten. Des weiteren ist die Öffnungskraft für die Membran relativ hoch, was u. a. auf die Bauweise und die Formgestaltung und Einspannlage der Membran zurückzuführen ist.

Solche Rückschlagventile finden in der Medizintechnik Verwendung, z. B. bei der sogenannten Parallelinfusion, bei der beispielsweise zu einer Infusionslösung, die dem Patienten mittels einer Infusionsflasche im Schwerkraftbetrieb zugeführt wird, über einen Nebenanschluß Medikamente in sehr geringen Mengen mittels einer Infusions- oder Spritzenpumpe beigegeben werden. Dabei muß vermieden werden, daß das Medikament aus der Infusionspumpe rückwärts in das Schwerkraftsystem fließt, welche Gefahr besteht, wenn der Zufluß der Infusionslösung zum Patienten unmerklich und möglicherweise vorübergehend blockiert ist. Die in Rede stehenden Rückschlagventile sind zu diesem Zweck so eingebaut, daß sie die beiden Systeme bei Normalfunktion in Zuflußrichtung zum Patienten miteinander verbinden, in Gegenrichtung aber voneinander trennen.

Die Anforderungen an ein Rückschlagventil dieser Art ist daher sehr hoch. So muß sichergestellt sein, daß das Rückschlagventil bereits bei einem durch Schwerkraft erzeugten Druck von ca. 0,01 bar öffnet, um einen ungehinderten Zufluß der Infusionslösung zu gewährleisten, andererseits muß es einem Schließdruck von ca. 6 bar widerstehen, der in dem Pumpsystem bei einer Störung auftreten kann. Dabei muß die genannte Öffnungsempfindlichkeit auch unmittelbar nach dem Einwirken des vollen Schließdruckes gewährleistet sein. Darüber hinaus muß es bei Förderraten von 0,1 ml/h sicher schließen, da dies die üblicherweise niedrigste Förderrate bei den verwendeten Spritzpumpen ist.

Ein weiteres, für den vorerwähnten Einsatz vorgesehenes Rückschlagventil ist Gegenstand der EP-A-01 82 045, die ein solches mit einer Dichtscheibe zeigt, die im Ruhezustand gegen die Einlaßöffnung durch eine zentrale Zweipunktstützung an diese angelegt gehalten wird und die durch den Infusionsdruck des Schwerkraftsystems eine bogenförmige Verformung erfährt, so daß die Infusionslösung die Dichtscheibe umfließen kann. Eine ähnliche Lösung ist dem DE-U-87 17 726 zu entnehmen. Schließlich zeigt noch die DE-C-26 05 348 ein Rückschlagventil, das durch Aufschwimmen einer Ventilscheibe funktioniert, wenn im Pumpsystem ein Überdruck auftritt.

Wie Versuche erwiesen haben, ist allen vorgenannten Rückschlagventilen gemeinsam, daß sie wenigstens eine der vorerwähnten Anforderungen nicht erfüllen. Prüfungsergebnisse sind in einem Artikel in der Zeitschrift "Biomedizinische Technik", Band 35, Heft 3/1990 veröffentlicht, welcher Artikel weitere Erfordernisse anspricht, die an solche Rückschlagventile zu stellen sind. Dabei ist insbesondere der durch das Rückschlagventil verursachte, den Durchfluß behindernde Strömungswiderstand hervorzuheben, der zu einer Durchflußminderung führt, die im Sinne einer exakten Regelbarkeit des Durchflusses möglichst klein sein sollte.

Die der Erfindung zugrundeliegende Aufgabe besteht daher in der Verbesserung eines Rückschlagventils der einleitend angeführten Art, das die genannten Erfordernisse optimal erfüllt und insbesondere bei einfacher Konstruktion in größerer Unabhängigkeit von Fertigungstoleranzen ein sicheres Öffnen und Schließen auch bei geringen Druckdifferenzen gewährleistet.

Die Lösung dieser Aufgabe ist in dem Patentanspruch 1 angegeben.

Bevorzugte Ausgestaltungen des erfindungsgemäßen Rückschlagventils sind in den Unteransprüchen aufgeführt.

Durch diese Lösung ist ein sicheres Arbeiten des Rückschlagventils bei geringeren Druckdifferenzen gewährleistet, und zwar auch dann, wenn insbesondere die funktionswichtigen Bauteile des Ventils durch größere Fertigungstoleranzen entstandene, an sich nicht akzeptierbare Abmessungsabweichungen aufweisen. Das ist darauf zurückzuführen, daß der Öffnungsdruck aus dem Einlaßkanal auf einen Bereich der Membran außerhalb des Dichtkraters wirkt, so daß eine große Öffnungskraft durch eine große Fläche der Membran bei kleiner Druckdifferenz erreicht wird. Daraus folgt, daß die Vorspannung der Membran groß sein kann und somit eine verbesserte Dichtsicherheit des Ventils erreicht wird. Des weiteren kann das Ventil mit größeren Fertigungstoleranzen hergestellt werden, ohne von seiner geforderten Wirksamkeit zu verlieren. Außerdem bleibt es in seiner Bauweise von einfacher Konstruktion.

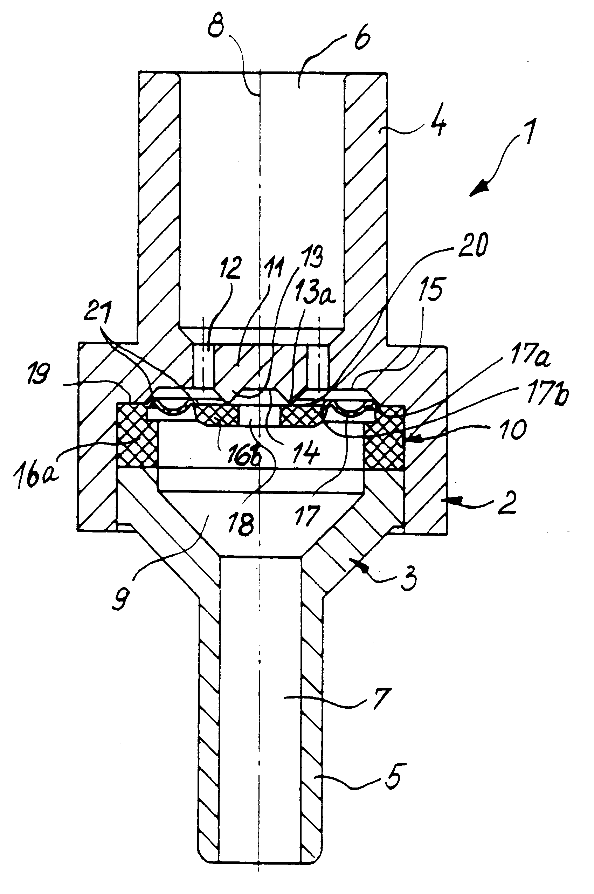

Ein Ausführungsbeispiel der Erfindung ist nachstehend anhand der Zeichnung naher erläutert, die in der einzigen Figur ein erfindungsgemäßes Rückschlagventil im Längsschnitt dargestellt zeigt.

Danach besteht das Rückschlagventil aus einem Gehäuse 1, das sich aus einem ersten und einem zweiten Gehäuseteil 2 bzw. 3 zusammensetzt. Die beiden Gehäuseteile 2 und 3 sind vorzugsweise im Kunststoffspritzverfahren aus einem geeigneten Thermoplast hergestellt und weisen je einen Schlauchstutzen 4 bzw. 5 auf, die einen Einlaßkanal 6 bzw. Auslaßkanal 7 umschließen, welche Kanäle zueinander fluchtend, d. h. mit einer gemeinsamen Achse 8 angeordnet sind. Die beiden Gehäuseteile 2 und 3 sind miteinander verbunden und begrenzen eine zentrale Kammer 9 von im wesentlichen zylindrischer Gestalt, in der eine Membran 10 aus einem hochflexiblen Werkstoff in ihrem Randbereich dichtend zwischen den Gehäuseteilen 2 und 3 eingespannt ist.

Die Kammer 9 ist beispielsweise durch eine ausströmseitig in dem Einlaßkanal 6 vorgesehene Querwand 11 begrenzt, die in ihrem Randbereich mit kleinen Durchlaßbohrungen 12 versehen ist, die auf einer um die Achse 8 konzentrisch verlaufenden Kreislinie angeordnet sind und den Einlaßkanal 6 mit der Kammer 9 verbinden Die Querwand 11 weist eine zentrale, kraterförmige Dichtungsausbildung 13, 14 auf, die innerhalb des Kreises für die Bohrungen 12 vorgesehen ist. Der Dichtkraterring 13 steht von der Querwand 11 in Richtung der Membran 10 vor, so daß eine innere Kraterfläche 14 und eine äußere Ringfläche 15 vertieft bestehen. Die Bohrungen 12 der Querwand 11 münden in die vertiefte Ringfläche 15 ein. Des weiteren ist eine ringförmige Einspannfläche 19 an dem Außenrandbereich der Membran 10 vorhanden, die mit der Dichtfläche 20 der Membran im wesentlichen in einer gemeinsamen Ebene liegt.

Die Membran 10 weist einen eingespannten, dickeren Randteil 16a auf und ist weiter mit einem scheibenförmigen, zentralen Dichtteil 16b versehen, welch letzterer mit dem Dichtkraterring 13 zur Erzielung einer öffenbaren Dichtung zusammenwirkt. Eine vorzugsweise sickenförmig profilierte, dünnere Ringwand 17 ist sowohl mit dem, eingespannten Ringteil als auch mit dem Dichtteil der Membran 10 verbunden. Dabei ist der Durchmesser des Dichtteiles 16b etwas größer als der Lochkreisdurchmesser für die Bohrungen 12. Schließlich weist der Dichtteil 16b eine kleine zentrale Bohrung 18 als Strömungsdurchlaß auf, die den innerhalb des Dichtkraterringes 13 befindlichen Raum mit der übrigen Kammer 9 verbindet, von der der Auslaßkanal 7 abgeht. Die Ringwand 17 ist beispielsweise im Querschnitt so gekrümmt ausgebildet, daß der Krümmungsscheitel von der Querwand 11 weggerichtet ist. Im Belastungsfall kann die Ringwand 17 jedoch so weit durchgedrückt werden, daß sie gegen die Ringfläche 15 zur Anlage kommt, wodurch sie hinsichtlich einer weiteren Durchbiegung entlastet ist. Weiterhin ist die Ringwand 17 so angeordnet, daß ihre Verbindungsstellen 17a, 17b an dem Randteil 16a bzw. an dem zentralen Dichtteil 16b der Membran 10 in einer gemeinsamen Ebene liegen, die wiederum mit der Dichtfläche 20 der Membran zusammenfällt.

Die dünne Ringwand 17 kann im Querschnitt auch dreieckig oder rechteckig geformt sein, vorteilhaft jedoch so, daß sich ein symmetrisches Wandprofil ergibt.

In einer weiteren Ausgestaltung der Membranringwand 17 kann diese auch so ausgebildet sein, daß ihre Randbereiche kurz vor ihrer Verbindung mit den anderen beiden Teilen 16a, 16b der Membran 10 im Querschnitt mit kleinem Radius der Querwand 11 zugekehrt gekrümmt ausgebildet sind, wie es mit der Bezugsziffer 21 deutlich gekennzeichnet ist. Die Ringwand 17 geht dann in die eigentliche, entgegengesetzt gekrümmte Sickenform über.

Der Dichtkraterring 13 weist einen Durchmesser auf, der nur wenig größer ist als der Strömungsdurchlaß 18 der Membran 10. Ferner ist der Dichtkraterring 13 im Querschnitt dreieckig profiliert, wobei sein spitz zulaufendes freies Ende die Dichtungsstelle 13a bildet.

Die Arbeitsweise des beschriebenen Rückschlagventils ist nachstehend anhand eines solchen in eine Einrichtung für die medizinische Parallelinfusion integrierten Rückschlagventils beschrieben. Dabei ist ein im Schwerkraftbetrieb arbeitendes (nicht gezeigtes) Infüsionssystem mit dem Einlaßkanal 6 verbunden, und es erfolgt eine weitere Medikamentenzuführung über ein (ebenfalls nicht gezeigtes) Druckinfusionssystem, das im Nebenschluß an den Auslaßkanal 7 angeschlossen ist.

Die Flüssigkeitssäule des Schwerkraftinfusionssystems lastet demnach durch die Bohrungen 12 hindurch auf der Membran 10 mit der Wirkung, daß ihr Dichtteil 16b, das eine vorbestimmte Vorspannung in Richtung auf den Dichtkraterring 13 aufweist, sich von diesem abhebt. Die Infüsionsflüssigkeit gelangt dadurch über die Bohrung 18 in dem Dichtteil 16b weiter in die Kammer 9 und dann in den Auslaßkanal 7. Dieser Zustand bleibt auch erhalten, wenn die Medikamentenzuführung aus dem Druckinfusionssystem ohne Drucksteigerung in dem zum Patienten führenden Infusionskatheter vonstattengeht. Tritt jedoch in dem Druckfusionssystem eine Drucksteigerung auf, so bewirkt das auch in dem Katheter eine Drucksteigerung, die in die Kammer 9 zurückwirkt und dort auf die gesamte Rückfläche der Membran 10 einwirkt. Die Membran sorgt aufgrund ihrer genannten Vorspannung sofort für eine schnelle und sichere Abdichtung in dem Rückschlagventil, wodurch ein unerwünschtes Eindringen von Medikamenten in das Schwerkraftinfusionssystem vermieden ist.

Bei weiterer Drucksteigerung kann die Membran 10 so verformt werden, daß die dünnere, sickenförmig profilierte Ringwand 17 sich an der Ringfläche 15 der Querwand 11 abstützend zur Anlage kommt, so daß die Ringwand bei hohen Drücken zugentlastet und eine Zerstörung der Membran 10 vermieden ist. Die angeführte Drucksteigerung in dem zum Patienten führenden Katheter kann zur Erzeugung eines Signals herangezogen werden, das dem Personal den gestörten Betriebszustand anzeigt.

Das beschriebene Rückschlagventil wird vorzugsweise auf medizinischem Gebiet eingesetzt. Es kann aber auch in Pumpen auf anderen Gebieten verwendet werden, wo die Betriebsbedingungen gleich oder ähnlich sind.

## Patentansprüche

1. Rückschlagventil, insbesondere für den Einsatz in Verbindung mit medizinischen Infusionsgeräten, aufweisend ein Gehäuse (1) mit einer zentralen Kammer (9), in die ein Einlaßkanal (6) und ein Auslaßkanal (7) mündet, welche Kanäle durch eine abdichtende Membran (10) mit Strömungsdurchlaß (18) voneinander trennbar sind, wobei die Membran einen im Gehäuse eingespannten Randteil (16a), einen zentralen Dichtteil (16b) und eine diese beiden Teile miteinander verbindende, dünne, eine Dichtungsvorspannung der Membran erzeugende Ringwand aufweist, wobei die Zuströmung aus dem Einlaßkanal (6) zu der Membran (10) ausserhalb der ringförmigen Dichtungsstelle an der Membran erfolgt und der Strömungsdurchlaß (18) der Membran innerhalb dieser ringförmigen Dichtungsstelle vorgesehen ist, dadurch gekennzeichnet, daß an der dem Einlaßkanal (6) zugekehrten Innenwandseite der Kammer (9) eine zentrale, kraterförmige Dichtungsausbildung (13, 14) als Dichtungsanlage für die Membran vorgesehen ist, daß die dünnere Ringwand (17) der Membran (10) im Querschnitt profiliert ist und ihre an den Randteil (16a) und den zentralen Dichtteil (16b) der Membran angrenzenden, freiliegenden Verbindungsstellen (17a, 17b) zusammen mit der Dichtungsfläche (20) des Dichtteiles (16b) in einer gemeinsamen Ebene liegen und daß die Einspannfläche (19) des Randteiles (16a) der Membran (10) im wesentlichen in der genannten gemeinsamen Ebene liegt.

2. Rückschlagventil nach Anspruch 1, dadurch gekennzeichnet, daß die Ringwand (17) im Querschnitt symmetrisch profiliert ist, z. B. Sicken-, Dreieck- oder Rechteckform aufweist, deren Scheitel vom Einlaßkanal (6) abgekehrt ist.

3. Rückschlagventil nach Anspruch 1, dadurch gekennzeichnet, daß in dem Einlaßkanal (6) ausströmseitig vor der Membran (10) eine Querwand (11) mit Durchlaßbohrungen (12) vorgesehen ist und daß die Querwand (11) eine äußere, sich um die kraterförmige Dichtungsausbildung (13, 14) erstreckende, vertiefte Ringfläche (15) als entlastende Stützfläche für die dünnere Ringwand (17) der Membran (10) im Belastungsfall aufweist.

4. Rückschlagventil nach Anspruch 1, dadurch gekennzeichnet, daß die beiden Randbereiche (21) der dünneren Membranringwand (17) kurz vor ihrer Verbindung mit den anderen beiden Teilen (16a, 16b) der Membran (10) im Querschnitt mit kleinem Radius der Querwand (11) zugekehrt gekrümmt ausgebildet sind und daß die Ringwand (17) dann in ihr entgegengesetzt ausgebildetes Querschnittsprofil übergeht.

5. Rückschlagventil nach Anspruch 1, dadurch gekennzeichnet, daß der Dichtkraterring (13) der kraterförmigen Dichtungsausbildung im Querschnitt dreieckig profiliert ist, wobei sein spitz zulaufendes freies Ende die Dichtungsstelle (13a) bildet.

## Claims

1. A check valve, in particular for application in combination with medical infusion apparatus, comprising a housing (1) with a central chamber (9) into which an inlet port (6) and an outlet port (7) run, said ports being separable from one another by way of a sealing membrane (10) having a flow opening (18), said membrane comprising a rim part (16a) clamped in the housing, a central sealing part (16b), and a thin annular wall (17) which connects these parts with one another and produces a sealing bias of the membrane, the incoming flow from the inlet port (6) to the membrane (10) occuring outside the ring shaped sealing location on the membrane and the flow opening (18) of the membrane being provided within this ring-shaped sealing location, characterised in that a central crater-shaped sealing formation (13,14) is provided as a sealing bearing for the membrane on the inner wall side of the chamber (9) facing the inlet port (6), that the thinner annular wall (17) of the membrane has a profiled cross-section and that its open connecting regions (17a, 17b) which border the rim part (16a) and the central sealing part (16b), together with the sealing surface (20) of the sealing part (16b) lie in a common plane and that the clamped surface (19) of the rim part (16a) of the membrane lies essentialy in said common plane.

2. A check valve according to claim 1, characterised in that the annular wall (17) has a symmetrically profiled cross-section, e.g. a crimped, triangular or rectangular shape, the apex of which facing away from the inlet port (6).

3. A check valve according to claim 1, characterised in that a transverse wall (11) with passage bores (12) is provided in the inlet port (6) on the oulet side in front of the membrane (10), and that the transverse wall (11) comprises an outer recessed annular surface (15) extending over the crater-shaped sealing formation (13,14) as a relieving support surface for the thinner annular wall (17) of the membrane (10) in the case of loading.

4. A check valve according to claim 1, characterised in that both of the rim regions (21) of the thinner annular wall (17) of the membrane, just in front of their connection to both the other parts (16a, 16b) of the membrane, are arched with the small radius facing the transverse wall (11) and that the annular wall (17) then changes into an oppositely formed cross-sectional profile.

5. A check valve according to claim 1, characterised in that the sealing crater annulus (13) of the crater-shaped sealing formation has a triangular cross-sectional profile, its pointed free end forming the sealing location (13a).

## Revendications

1. Soupape antiretour, destinée en particulier à une utilisation en liaison avec des appareils médicaux de perfusion, présentant un corps (1) muni d'une chambre centrale (9), dans laquelle débouchent un canal d'entrée (6) et un canal de sortie (7), qui peuvent être isolés l'un de l'autre par une membrane (10) réalisant une étanchéité et comportant un passage d'écoulement (18), la membrane présentant une partie périphérique (16a) enserrée dans le corps, une partie centrale de fermeture étanche (16b) et une paroi annulaire mince qui relie l'une à l'autre ces deux parties et engendre une précontrainte de la membrane dans le sens de la fermeture étanche, l'écoulement qui afflue du canal d'entrée (6) vers la membrane (10) se faisant, au niveau de la membrane, à l'extérieur de la zone de contact étanche annulaire, tandis que le passage d'écoulement (18) de la membrane est prévu à l'intérieur de cette zone de contact étanche annulaire, caractérisée en ce que sur la face, située du côté du canal d'entrée (6), de la paroi intérieure de la chambre (9), il est prévu une structure d'étanchéité centrale en forme de cratère (13, 14) constituant un appui de contact étanche pour la membrane, en ce que la paroi annulaire amincie (17) de la membrane (10) est profilée en coupe transversale et ses zones de liaison dégagées (17a, 17b), contiguës à la partie périphérique (16a) et à la partie de fermeture étanche centrale (16b) de la membrane, sont situées dans un plan commun, conjointement avec la surface de contact étanche (20) de la partie de fermeture étanche (16b), et en ce que la surface enserrée (19) de la partie périphérique (16a) de la membrane (10) est située, pour l'essentiel, dans ledit plan commun.

2. Soupape antiretour selon la revendication 1, caractérisée en ce que la paroi annulaire (17) a, en coupe transversale, un profil symétrique, et présente, par exemple, la forme d'une moulure, d'un triangle ou d'un rectangle, dont le sommet est dirigé à l'opposé du canal d'entrée (6).

3. Soupape antiretour selon la revendication 1, caractérisée en ce que, dans le canal d'entrée (6), il est prévu, du côté de sa sortie et en amont de la membrane (10), une paroi transversale (11) comportant des forures de passage (12) et en ce que la paroi transversale (11) présente une surface annulaire extérieure en renfoncement (15), s'étendant autour de la structure d'étanchéité en forme de cratère (13, 14) et constituant une surface d'appui pour le soutien de la paroi annulaire amincie (17) de la membrane (10) dans le cas d'une sollicitation.

4. Soupape antiretour selon la revendication 1, caractérisée en ce que les deux régions de bord (21) de la paroi annulaire amincie (17) de la membrane, juste en-deçà de leur jonction aux deux autres parties (16a, 16b) de la membrane (10), sont réalisées, en coupe transversale, avec une courbure de petit rayon, dirigée vers la paroi transversale (11), et en ce que la paroi annulaire (17) prend ensuite son profil en coupe transversale, formé en sens oppose.

5. Soupape antiretour selon la revendication 1, caractérisée en ce que l'anneau d'étanchéité (13), définissant le cratère, de la structure d'étanchéité en forme de cratère, présente un profil triangulaire en coupe transversale, son extrémité libre convergente en pointe constituant la zone de contact étanche (13a).
